# EUROPEAN PATENT APPLICATION

(11) **EP 3 698 802 A1**
(43) Date of publication of application: **26.08.2020**
(21) Application number: 18867476.6
(22) Date of filing: 17.10.2018
(51) Int. Cl.: A61K 35/28, A61K 35/545, A61P 19/04

(54) **PLURIPOTENT STEM CELLS INDUCING OSTEOCHONDRAL REPAIR**

(30) Priority: 17.10.2017 US 201762573500 P
(71) Applicant: Hiroshima University, Higashihiroshima-shi, Hiroshima 739-8511 (JP); Tohoku University, Sendai-shi, Miyagi 980-8577 (JP)
(72) Inventor: KAMEI, Naosuke, Higashihiroshima-shi Hiroshima 739-8511 (JP); DEZAWA, Mari, Sendai-shi Miyagi 980-8577 (JP); OCHI, Mitsuo, Higashihiroshima-shi Hiroshima 739-8511 (JP)
(74) Representative: AWA Sweden AB
(86) International application number: PCT/JP2018/038687
(87) International publication number: WO 2019/078262

(57) **Abstract**

Multilineage-differentiating stress enduring (Muse) cells are stage-specific embryonic antigen-3 (SSEA-3) positive cells that exist in mesenchymal stem cell (MSC) populations. Muse cells have the pluripotency to differentiate into all germ layers as embryonic stem cells. The purpose of the present study is to investigate the efficacy of Muse cell transplantation for repairing osteochondral defects. Muse cells were isolated from human bone marrow MSCs. As osteochondral defects, the patellar grooves of immunodeficient rats were injured. Next, cells were injected into the mice so that the animals were divided into the following 3 groups: a control group to which PBS was injected; a non-Muse group to which 5 x 10⁴ SSEA-3 negative non-Muse cells were injected; and a Muse group to which 5 x 10⁴ SSEA-3 positive Muse cells were injected. In the Muse group, repaired tissues with almost smooth and homogenous surface were observed 12 weeks after the treatment, while no repaired tissue was found in the control and non-Muse groups. Histological evaluation indicates that the Muse group showed better repair in the osteochondral defect sites 4 and 12 weeks after the treatment, compared to the other groups. Thus, Muse cells are likely a novel promising cell source for treating osteochondral defects.

## Description

### FIELD

The present invention relates to a cell preparation and/or pharmaceutical composition used in regenerative medicine. More particularly, the present invention relates to a cell preparation or pharmaceutical composition containing pluripotent stem cells effective for treatment or repair of osteochondral damage.

### BACKGROUND

Cartilage lesions cause joint disorders due to limited self-repair ability and a decrease in joint function, and are equivalent to serious disorders particularly in elderly patients (NPL1) [1].

Although several clinical studies using methods such as bone marrow stimulation technology or osteochondral transplantation have been conducted in an attempt to improve cartilage repair, their success has been limited. Brittberg, et al. conducted first generation cell therapy referred to as autologous chondrocyte transplantation (ACI) in 1994 (NPL2) [2]. Ochi, et al. demonstrated a favorable clinical outcome by modifying ACI by combining atelocollagen gel with chondrocytes (NPL3) [3]. However, success remained limited due to the morbidity of intact cartilage, dedifferentiation and second stage surgical treatment.

Recently in the field of regenerative medicine, research has been conducted on cell therapy using stem cells against various diseases, and embryonic stem cells (ES cells), neural stem/progenitor cells (NSPC), induced pluripotent stem cells (iPS) and umbilical cord blood stem cells (UCBC) are known as stem cells that are expected to have the potential for clinical applications.

Mesenchymal stem cells (MSC) have been isolated from adults and are known to have the ability to differentiate into, for example, bone, cartilage, adipocytes and skeletal muscle (NPL4,5). However, MSC comprise a cell group that contains various cells and the actual state of the differentiation ability thereof is not fully understood resulting in considerable variation in therapeutic efficacy. Although iPS cells have been reported to be adult-derived pluripotent stem cells (PTL1), in addition to the establishment of iPS cells requiring the extremely complex procedure of introducing a specific gene or specific compound into a mesenchymal cell fraction of skin fibroblasts, since iPS cells have a potent tumorigenic ability, extremely high hurdles must be overcome for their clinical application.

It has become easy to isolate mesenchymal stem cells (MSC) during the past 10 years, and since MSC can be accessed extremely easily from various tissues and can be grown extremely rapidly, they are widely used as cell-based treatment methods for clinical application. MSC can also widely differentiate into all three germ layers (NPL6) [4].

It has been determined from research conducted by M. Dezawa, one of the inventors of the present invention, that multilineage-differentiating stress-enduring (Muse) cells, which are present in mesenchymal cell fractions, can be obtained without requiring an induction procedure, and express stage-specific embryonic antigen-3 (SSEA-3) as surface antigen, are responsible for the pluripotency demonstrated by mesenchymal cell fractions, and have the potential to be able to be applied to disease treatment targeted at tissue regeneration. Muse cells have also been determined to be able to be concentrated by stimulating mesenchymal cell fractions with various types of stress (PTL2, NPL7).

However, there have been no examples clearly demonstrating that Muse cells can be used for improvement and/or treatment of brain damage accompanying fetal growth retardation or that anticipated clinical effects are obtained.

### [CITATION LIST]

### [PATENT LITERATURE]

[PTL 1] JP 4183742 B
[PTL 2] WO 2011/007900 A

### [NON PATENT LITERATURE]

[NPL 1] A. Mobasheri, et al., Histol. Histopathol., vol. 24, p.347-366 (2009)
[NPL 2] M. Brittberg, et al., N. Engl. J. Med., vol. 331, p.889-895 (1994)
[NPL 3] M. Ochi, et al., J. Bone. Joint Surg. Br., vol. 84, p.571-578 (2002)
[NPL 4] M. Dezawa, et al., J. Clin. Investi., vol. 113, p.1701-1710 (2004)
[NPL 5] M. Dezawa, et al., Science, vol. 309, p.314-317 (2005)
[NPL 6] K. Tamai, et al., Proc. Natl. Acad. Sci. USA, vol. 108, p.6609-6614 (2011)
[NPL 7] Wakao,S, et al., Proc. Natl. Acad. Sci. USA, Vol. 108, p.9875-9880 (2011)

### SUMMARY

### [TECHNICAL PROBLEM]

An object of the present invention is to provide a novel medical application using pluripotent stem cells (Muse cells) in regenerative medicine. More specifically, an object of the present invention is to provide a cell preparation and/or pharmaceutical composition containing Muse cells for repairing osteochondral damage.

### [SOLUTION TO PROBLEM]

The inventors of the present invention found that, as a result of injecting or administering Muse cells in an immunodeficient rat model, the Muse cells locally accumulate at sites of osteochondral damage, differentiate into chondrocytes at the damaged site, and are capable of repairing that osteochondral damage, thereby leading to completion of the present invention.

Specifically, the present invention provides as follows.
[Item 1] A cell preparation for treating or repairing osteochondral damage, comprising: pluripotent stem cells positive for SSEA-3 isolated from a body mesenchymal tissue or cultured mesenchymal cells.
[Item 2] The cell preparation according to [Item 1], wherein the cell preparation contains a cell fraction wherein the pluripotent stem cells positive for SSEA-3 have been concentrated by external stress stimulation.
[Item 3] The cell preparation according to [Item 1] or [Item 2], wherein the pluripotent stem cells are CD105-positive.
[Item 4] The cell preparation according to any of [Item 1] to [Item 3], wherein the pluripotent stem cells are CD117-negative and CD146-negative.
[Item 5] The cell preparation according to any of [Item 1] to [Item 4], wherein the pluripotent stem cells are CD117-negative, CD146-negative, NG2-negative, CD34-negative, vWF-negative and CD271-negative.
[Item 6] The cell preparation according to any of [Item 1] to [Item 5], wherein the pluripotent stem cells are CD34-negative, CD117-negative, CD146-negative, CD271-negative, NG2-negative, vWF-negative, Sox10-negative, Snail-negative, Slug-negative, Tyrp1-negative and Dct-negative.
[Item 7] The cell preparation according to any of [Item 1] to [Item 6], wherein the pluripotent stem cells are pluripotent stem cells having all of the properties indicated below:
   (i) low or absent telomerase activity;
   (ii) ability to differentiate into any of three germ layers;
   (iii) absence of demonstration of neoplastic proliferation; and,
   (iv) self-renewal ability.
[Item 8] The cell preparation according to any of [Item 1] to [Item 7], wherein the pluripotent stem cells have the ability to accumulate at sites of osteochondral damage.
[Item 9] The cell preparation according to any of [Item 1] to [Item 8], wherein the pluripotent stem cells have the ability to differentiate into chondrocytes.

The present invention is capable of repairing osteochondral damage, and particularly subchondral bone covered by fibrous tissue, by an osteochondral tissue regeneration mechanism by which Muse cells differentiate into chondrocytes at a damaged site as a result of directly or intravenously administering Muse cells to the damaged site in a subject suffering from osteochondral damage.

### BRIEF DESCRIPTION OF DRAWINGS

FIG. 1 depicts macroscopic findings of repaired tissue in a control group, non-Muse group and Muse group at 4 and 12 weeks after treatment. In the Muse group after 12 weeks, the damaged site was completely replenished by white tissue to the same degree as normal tissue.
FIG. 2 indicates a macroscopic evaluation system, with an improving trend being observed in the Muse group at 4 weeks after treatment. After 12 weeks, the Muse group exhibited significantly better results than the other groups (*P<0.05).
FIG. 3A depicts histological evaluations of repaired tissue using safranin O/fast green stain at 4 weeks and 12 weeks. FIGS. 3B and 3C indicate images obtained by H & E staining at 12 weeks. The scale bars represent 500 µm in FIGS. 3A and 3B and 100 µm in FIG. 3C.
FIG. 4 depicts the results of carrying out histological scoring using the Sellers scale at 4 weeks and 12 weeks, with a histologically significant difference being observed in the Muse group differing from both the non-Muse group and control group (*P<0.05).

### DESCRIPTION OF EMBODIMENTS

The present invention relates to a cell preparation or pharmaceutical composition containing SSEA-3-positive pluripotent stem cells (Muse cells) for treating or repairing osteochondral damage.

### 1. Applicable Diseases

The present invention can be used to treat, repair or alleviate diseases or symptomatic conditions relating to osteochondral damage such as osteoarthritis, traumatic cartilage damage, rheumatoid arthritis or cancer by using a cell preparation or pharmaceutical composition containing SSEA-3-positive pluripotent stem cells (Muse cells). In the present invention, "osteochondral damage" refers to damage to bone and/or cartilage attributable to the aforementioned diseases as well as damage occurring as a result of an accident or surgical procedure, although not limited thereto. "Damage" refers to a change in the normal structure or function, has the same meaning as the commonly used term, and can be used synonymously with disorder, degeneration, trauma or defect.

When used in the present invention, "bone" mainly refers to calcium and phosphoric acid components that have been deposited in the form of hydroxyapatite or collagen (and mainly type I collagen), osteocytes (such as osteoblasts, osteocytes and osteoblasts), and calcified (mineralized) connective tissue including bone marrow formed within true endochondral bone. Bone tissue significantly differs from other tissue (including cartilage tissue). More specifically, bone tissue is vascularized tissue composed from cells and biphasic media (including mineralized and inorganic components (mainly hydroxyapatite crystals) and organic components (mainly type I collagen). Glycosaminoglycans compose less than 2% of these organic components and less than 1% of biphasic media per se or bone tissue per se. The collagen present in bone tissue is present in an extremely organized parallel arrangement in comparison with cartilage tissue.

When used in the present invention, "cartilage" refers to connective tissue containing chondrocytes, chondrocyte-like cells, intercellular substances (such as type I, type II, type IX and type XI collagen), proteoglycans (such as chondroitin sulfate proteoglycans, keratan sulfate proteoglycans and dermatan sulfate proteoglycans), and other proteins. Cartilage includes articular cartilage and non-articular cartilage.

"Articular cartilage" refers to non-mineralized connective tissue that covers the connecting surface of bone within a joint and fulfills the function as a friction-reducing joint between two opposing bone surfaces. Articular cartilage allows bones move without making direct contact. Articular cartilage partially obtains nutrients from blood vessels of the adjacent periosteum and blood vessels of the bone covered thereby. Articular cartilage contains type II and type IX collagen along with various proteoglycans, but does not contain X type collagen that is related to endochondral osteogenesis. Articular cartilage covers the surface of bone and the bone beneath the cartilage is referred to as subchondral bone. Damage extending to cartilage and subchondral bone is referred to as osteochondral damage (cartilage full thickness defect), while damage limited to the cartilage layer is referred to as cartilage damage (cartilage partial defect). In the present invention, "cartilage damage" includes the aforementioned "osteochondral damage" and "cartilage damage".

"Non-articular cartilage" refers to cartilage not covering a joint surface and includes fibrocartilage (fibrous disc, fibrocartilage disc, connective fibrocartilage and periarticular fibrocartilage) and soft cartilage. In fibrocartilage, a micropolysaccharide network is combined with protruding collagen bundles and chondrocytes are scattered over a wider range than hyaline cartilage or articular cartilage. The fibrous disc is exposed to impacts and is frequency found in moving joints (such as the meniscus of the knee). Examples of such joints include, but are not limited to, the temporomandibular joint, sternoclavicular joint and knee joint. Secondary articulation is formed by the meniscus of fibrocartilage. This fibrocartilage disc, which closely adheres to both opposing surfaces, is composed of concentric rings of fibrous tissue having thin layers of cartilage interposed there between. An example this fibrocartilage disc is an intervertebral disc of the spinal cord. Connective fibrocartilage is interposed between the bone surfaces of these joints and is able to move slightly between a vertebral body and between the pubic bone. Periarticular fibrocartilage surrounds the periphery of several articular cavities.

### 2. Cell preparation

### (1) Pluripotent stem cells

The pluripotent stem cells to be used in the cell preparation and pharmaceutical composition of the present invention are typically cells that reside in the human body. The cells which are named "Muse (Multilineage-differentiating Stress Enduring) cells" were discovered by Prof. Dezawa, one of the present inventors. Muse cells can be obtained from bone marrow aspirates and adipose tissue (Ogura, F., et al., Stem Cells Dev., Nov 20, 2013 (Epub) (published on Jan 17, 2014)) or from skin tissue such as dermal connective tissue, and they are widely dispersed throughout the connective tissue of various organs. The cells have the properties of both pluripotent stem cells and mesenchymal stem cells, and are identified as cells double-positive for the cell surface markers "SSEA-3 (Stage-specific embryonic antigen-3)" and "CD105". Therefore, Muse cells or cell populations containing Muse cells, for example, can be isolated from body tissue using these antigen markers. Muse cells are also stress-tolerant, and can be concentrated from mesenchymal tissue or cultured mesenchymal cells by different types of stress treatments. A cell fraction in which Muse cells were enriched into high content by stress treatment may be used as the cell preparation of the present invention. The methods of separation and identification of Muse cells, and their features, are disclosed in detail in International Patent Publication No. WO2011/007900. Also, as reported by Wakao et al. (2011, ibid.), all of Muse cells isolated from the bone marrow- or skin-derived mesenchymal cells were both positive for SSEA-3and CD105. According to the present invention, in cases where Muse cells are isolated from mesenchymal tissue of a body or cultured mesenchymal stem cells SSEA-3 can be simply used as the antigen marker for the purpose of isolating Muse cells. Throughout the present specification, pluripotent stem cells (Muse cells) or a cell population containing Muse cells, which were isolated from mesenchymal tissue of a body or cultured mesenchymal tissue by using SSEA-3 as the antigen marker, and which can be used in the cell preparation for treating osteochondral damage (including sequela thereof), may be referred to simply as "SSEA-3 positive cells". Also throughout the present specification, "non-Muse cells" refers to cells that are present in mesenchymal tissue of a body or cultured mesenchymal tissue, and are the remainder of "SSEA-3 positive cells".

In brief, Muse cells or a cell population containing Muse cells can be isolated from body tissue (for example, mesenchymal tissue) using only antibody for the cell surface marker SSEA-3, or using antibodies for both SSEA-3 and CD105. The term "body" here means "mammalian body". According to the present invention, the "body" does not include a fertilized ovum or an embryo at a developmental stage before the blastocyst stage, but it does include an embryo at the developmental stage from the blastocyst stage onward, including the fetus or blastocyst. The mammal is not limited to a certain species and may be a primate such as human or monkey, a rodent such as a mouse, rat, rabbit or guinea pig, or a cat, dog, sheep, pig, cow, horse, donkey, goat or ferret. The Muse cells to be used in the cell preparation and pharmaceutical composition of the present invention are clearly distinguished from embryonic stem cells (ES cells) or iPS cells in terms of direct separation from body tissue by using a specified marker. The term "mesenchymal tissue" refers to tissue from the bone, synovial membrane, fat, blood, bone marrow, skeletal muscle, dermis, ligament, tendon, dental pulp, umbilical cord or umbilical cord blood, or tissues present in various organs. For example, the Muse cells may be obtained from the bone marrow or skin or adipose tissue. Preferably, mesenchymal tissue of a body is harvested and the Muse cells are isolated from the tissue and used. The separating means mentioned above may be used to separate Muse cells from cultured mesenchymal cells such as fibroblasts or bone marrow-derived MSCs. The Muse cells to be used for the cell preparation and pharmaceutical composition of the present invention may be either autologous or allogenic with respect to the recipient.

As mentioned above, Muse cells or a cell population containing Muse cells can be isolated from body tissue by using SSEA-3 positivity, or double positive for SSEA-3 and CD105, as indicators, but human adult skin is known to include various types of stem cells and progenitor cells. However, Muse cells are not identical to these cells. Such stem cells and progenitor cells include skin-derived precursors (SKP), neural crest stem cells (NCSC), melanoblasts (MB), perivascular cells (PC), endothelial precursor cells (EP) and adipose-derived stem cells (ADSC). Muse cells can be separated out as being "non-expressing" for the markers unique to these cells. More specifically, Muse cells can be separated by using non-expression for at least one, and for example, 2, 3, 4, 5, 6, 7, 8, 9, 10 or 11 among 11 markers selected from the group consisting of CD34 (EP and ADSC marker), CD117 (c-kit) (MB marker), CD146 (PC and ADSC marker), CD271 (NGFR) (NCSC marker), NG2 (PC marker), vWF factor (von Willebrand factor) (EP marker), Sox10 (NCSC marker), Snail (SKP marker), Slug (SKP marker), Tyrp1 (MB marker) and Dct (MB marker). As a non-limitative example, non-expression of CD117 and CD146 may be used as the indicator for separation, non-expression of CD117, CD146, NG2, CD34, vWF and CD271 may be used as the indicator, or non-expression of all of the aforementioned 11 markers may be used as the indicator for separation.

The Muse cells having the aforementioned features to be used for the cell preparation and pharmaceutical composition of the present invention may have at least one property selected from the group consisting of the following:
(i) telomerase activity at low or under detection level;
(ii) having the ability to differentiate into cells of the three germ layers;
(iii) exhibiting no tumorigenic proliferation; and
(iv) having self-renewal ability.
According to one aspect of the present invention, the Muse cells to be used for the cell preparation and pharmaceutical composition of the present invention have all of these properties. As regards (i), "telomerase activity at low or under detection level", this refers to low level or the level under detection limit telomerase activity when using a TRAPEZE XL telomerase detection kit (Millipore), for example. "Low" telomerase activity is, for example, either telomerase activity on the same level as somatic cells such as human fibroblasts, or telomerase activity of 1/5 and preferably no greater than 1/10 of that of Hela cells. In regard to (ii), the Muse cells have the ability to differentiate into the three germ layers (endoderm, mesoderm and ectoderm) in vitro and in vivo, and by induction culturing in vitro, for example, they can differentiate into hepatocytes, neurons, skeletal muscle cells, smooth muscle cells, osteocytes or adipocytes. They may also exhibit the ability to differentiate into the three germ layers in the case of transplanting in vivo into the testes. They also have the ability to migrate to and engraft onto damaged organs (heart, skin, spine, liver, muscle, etc.), by administration into the body via intravenous injection, and differentiate into specific cells of the corresponding tissue. In regard to (iii), the Muse cells have the property of proliferating at a rate of about every 1.3 days in suspension culture, and growing in suspension culture from a single cell to form an embryoid-like cell mass, slow down the growth at about 14 days; however, when the embryoid-like cell mass is transferred to adhesion culture, cell growth resumes and the proliferated cells spread out from the cell mass. They also have the property of not generating teratomas at least for 6 months after transplantation into the testes. In regard to (iv), Muse cells have self-renewal (auto-replicating) ability. The term "self-renewal" means that cells in the embryoid-like cell mass obtained by culturing a single Muse cell in suspension culture can be confirmed to differentiate into cells of all 3 germ layers, and also that when a single cell from the embryoid-like cell mass is again carried into a suspension culture, it forms a next generation embryoid-like cell mass, and reproduce differentiation into three germ layers as well as embryoid-like cell mass in the suspension culture can be confirmed. Self-renewal may be observed once or as several repeated cycles.

In addition, a cell fraction containing Muse cells to be used in the cell preparation of the present invention may be a cell fraction having the SSEA-3 positive and CD105-positive pluripotent stem cells concentrated, obtained by a method of applying external stress treatment to mesenchymal tissue of a body or cultured mesenchymal cells, causing the cells other than the external stress-resistant cells to die, and recovering the surviving cells, the cell fraction having at least one and preferably all of the following properties.
(i) SSEA-3 positivity;
(ii) CD105-positivity;
(iii) telomerase activity at low or under detection level;
(iv) having the ability to differentiate into cells of the three germ layers;
(v) exhibiting no neoplastic proliferation activity; and
(vi) having self-renewal ability.

The external stress may be any one or a combination of: protease treatment, culturing in a low oxygen concentration, culturing under low-phosphate conditions, culturing with low serum concentration, culturing under low nutritive conditions, culturing under exposure to heat shock, culturing at low temperature, freezing treatment, culturing in the presence of a hazardous substance, culturing in the presence of active oxygen, culturing under mechanical stimulation, culturing with agitating treatment, culturing with pressure treatment, or physical impact. For example, the treatment time with a protease is preferably a total of 0.5 to 36 hours to apply external stress to the cells. The protease concentration may be the concentration used when the cells adhering to a culture vessel are detached, when the cell mass is dispersed into individual cells, or when individual cells are recovered from tissue. The protease is preferably a serine protease, aspartic acid protease, cysteine protease, metalloprotease, glutamic acid protease or N-terminal threonine protease. The protease is also preferably trypsin, collagenase or dispase.

### (2) Preparation and Use of Cell Preparation

The cell preparation of the present invention, although not limited thereto, is obtained by suspending Muse cells or a cell population containing Muse cells obtained in the aforementioned (1) in physiological saline or a suitable buffer (such as phosphate-buffered physiological saline). In this case, in the case the number of Muse cells isolated from autogenous or allogenous tissue is low, cells may be cultured prior to cell transplant and allowed to proliferate until a prescribed cell concentration is obtained. Furthermore, as has been previously reported (International Publication No. WO 2011/007900), since Muse cells do not undergo neoplastic transformation, there is little likelihood of the cells becoming malignant even if cells recovered from biological tissue are contained that have still not differentiated, thereby making them safe. In addition, although there are no particular limitations thereon, culturing of recovered Muse cells can be carried out in an ordinary growth medium (such as minimum essential medium-α (a-MEM) containing 10% bovine calf serum). More specifically, a solution containing a prescribed concentration of Muse cells can be prepared by selecting media, additives (such as antibiotics and serum) and the like suitable for the culturing and proliferation of Muse cells with reference to the aforementioned International Publication No. WO 2011/007900. In the case of administering the cell preparation of the present invention to a human, roughly several milliliters of bone marrow aspirate are collected from human ilium, and after isolating Muse cells by using an antigen marker for SSEA-3 as an indicator, the cells are allowed to proliferate by culturing for an appropriate amount of time until an effective therapeutic dose is reached, followed by preparing autologous or allogenic Muse cells in the form of a cell preparation. Alternatively, for instance, Muse cells are isolated by using an antigen marker for SSEA-3 as an indicator, and then after the cells are allowed to proliferate by culturing for an appropriate amount of time until an effective therapeutic dose is reached, autologous or allogenic Muse cells can be prepared as a cell preparation.

In addition, when using the cell preparation of Muse cells, dimethylsulfoxide (DMSO) or serum albumin for protecting the cells, or antibiotics and the like for preventing contamination and growth of bacteria, may also be contained in the cell preparation. Moreover, other pharmaceutically allowable components (such as a carrier, vehicle, disintegrating agent, buffer, emulsifier, suspending agent, soothing agent, stabilizer, storage agent, preservative or physiological saline), or cells or components other than Muse cells contained in mesenchymal cells, may also be contained in the cell preparation. A person with ordinary skill in the art is able to add these factors and pharmaceutical agents to a cell preparation at suitable concentrations. In this manner, Muse cells can be used in the form of a pharmaceutical composition containing various types of additives.

The number of Muse cells contained in the cell preparation prepared in the manner described above can be suitably adjusted in consideration of the gender, age and body weight of the subject, disease state and state in which the cells are used so as to obtain the desired effect in treatment of osteochondral damage (such as regeneration of bone and cartilage, disappearance of various diseases related to osteochondral damage, etc.). In Examples to be subsequently described, a rat osteochondral defect model with a part of the femur missing, and various types of effects of transplanting Muse cells were examined. Extremely superior effects were obtained by intra-articular administration of SSEA3-positive cells to the rats weighing about 200g to 300g at 5 x 10⁴ cells/animal. On the basis of this result, superior effects can be expected to be obtained by administering 1.6 to 2.5 x 105 cells/kg per individual mammal based on body weight. Here, examples of individuals include, but are not limited to, rats and humans. In addition, the cell preparation of the present invention may be administered a plurality of times (such as 2 to 10 times) at a suitable interval (such as twice per day, once per day, twice per week, once per week, once every two weeks, once every one month, one every two months, once every six months) until the desired therapeutic effect is obtained. Thus, although dependent upon the status of the subject, the therapeutically effective dose is preferably administered, for example, 1 to 10 times at 1 x 10³ cells to 2 x 10⁷ cells per individual. Although there are no particular limitations thereon, examples of total individual doses include 1 x 10³ cells to 2 x 10⁸ cells, 1 x 10⁴ cells to 1 x 10⁸ cells, 2 x 10⁴ cells to 5 x 10⁷ cells, 5 x 10⁴ cells to 5 x 10⁷, and 1 x 10⁵ cells to 1 x 10⁷ cells. Further, the cell preparation of the present invention is not particularly limited to, but may be directly administered to osteochondral damage sites (for example, a joint, a femur, etc.), or may be intravenously administered.

### 3. Preparation of Rat Osteochondral Defect Model

In the present description, a rat osteochondral defect model can be constructed and used in order to study the therapeutic effect of the cell preparation of the present invention on osteochondral damage. Examples of rats used as the model typically include, but are not limited to, immunodeficient (such as F344/NJcl-rnu/rnu) Wistar rats and Sprague-Dawley (SD) rats. In the examples to be subsequently described, a rat model was used in which an osteochondral defect was formed in the femur bones of the rats with a metal drill having a spherical tip measuring 1 mm in diameter. The cell preparation of the present invention is in a heterologous relationship with respect to the rats administered the preparation since the preparation consists of human-derived Muse cells. Normally in an experiment in which heterologous cells are administered to a model animal, an immunosuppressant (such as cyclosporine) is administered either prior or simultaneous to administration of the heterologous cells in order to suppress a rejection reaction by the heterologous cells within the body.

### 4. Therapeutic Effect of Muse Cells

In embodiments of the present invention, the cell preparation of the present invention is able to restore the function of the bone and cartilage of a patient with osteochondral damage to normal. When used in the present description, "restoration" of bone and cartilage function refers to treatment, repair and alleviation of various types of functional disorders accompanying osteochondral damage (including defects), and as an example thereof, refers to alleviation of bone and cartilage damage to a degree that does present a problem during the course of daily life. "Restoration" of bone and cartilage function also refers to a functional disorder caused by osteochondral damage being improved, alleviated or eliminated and returning to the state prior to the osteochondral damage. Histological scoring according to Sellers using safranin O/fast green stain can typically be used to carry out evaluation of restoration of bone and cartilage function as a histological evaluation, although not limited thereto[8].

Although the following provides a more detailed explanation of the present invention through examples thereof, the present invention is not limited by these examples.

### EXAMPLES

Methods relating to the animal experiments of this study were carried out in accordance with the animal experimentation guidelines of Hiroshima University. All protocols were implemented after being approved by the Laboratory Animal Science Research Facility Committee of the Graduate School of Biomedical and Health Sciences of Hiroshima University.

### EXAMPLE 1: Cell Preparation

Human bone marrow MSC (hBMSC, Lonza, Basel, Switzerland) were purchased and cultured in Eagle's minimum essential medium (a-MEM) containing 10% fetal bovine serum (FBS), 0.1 mg/ml kanamycin and 1% Glutamax (Thermo Fisher Scientific, Waltham, MA) at 37°C in 5% CO₂. After the hBMSC reached 90-100% confluency, the cells were subcultured using 0.25% trypsin-ethylenediamine tetraacetate at a ratio of 1:2. Subculturing was carried out according to the protocol of Kuroda et al [5]. Simply stated, the hBMSC were separated into Muse cells (SSEA-3⁺) and non-Muse cells (SSEA-3⁻) corresponding to the presence or absence of expression of SSEA-3. The hBMSC were incubated with SSEA-3 antibody (1:100, Merck Millipore, Darmstadt, Germany) and detected with anti-rat IgM conjugated with allophycocyanin (Jackson Immuno Research, West Grove, PA) in diluted antibody to classify according to FACS Aria II (Becton Dickinson, Franklin Lakes, NJ), which is a special order research project.

### EXAMPLE 2: Introduction of Cells into Osteochondral Defect Site

The present example was carried out with 16 10-week-old immunodeficient rats (32 knees) (F344/NJcl-rnu/rnu). Osteochondral defects (diameter: 2 mm, depth: 2 mm) were formed on the left and right sides within the patellar groove of the femur using a commercially available metal drill having a spherical tip measuring 1 mm in diameter. The rat knees were unevenly distributed among three groups immediately after closing the knee joint (Table 1). The control group was injected with PBS, the non-Muse group was intraarticularly injected with non-Muse cells (5 × 10⁴ cells), and the Muse group was intraarticularly injected with Muse cells (5 × 10⁴ cells). The cells were suspended in 50 µl of PBS.

### [Table 1]

**Table 1**

| Time Point | Control Group | Non-Muse Group | Muse Group | Total |
|---|---|---|---|---|
| 4 weeks | 5 | 6 | 5 | 16 |
| 12 weeks | 4 | 6 | 6 | 16 |
| Total | 9 | 12 | 11 | 32 |

### EXAMPLE 3: Macroscopic and Histological Evaluations

### (1) Macroscopic Evaluation Method

The rats were sacrificed at 4 weeks and 12 weeks after treatment by intraperitoneal injection of a lethal dose of pentobarbital sodium. The femoral condoyle was evaluated macroscopically using the macroscopic scoring system according to Wayne et al [6][7]. A score of 14 indicates the best result while a score of 0 indicates the poorest result (Table 2).

Table 2 Macroscopic Scoring System

**[Table 2-1]**

| (I) Coverage | |
|---|---|
| Replenishment of >75% | 4 |
| Replenishment of 50-75% | 3 |
| Replenishment of 25-50% | 2 |
| Replenishment of <25% | 1 |
| No replenishment | 0 |

**[Table 2-2]**

| (II) Color of New Cartilage | |
|---|---|
| Normal | 4 |
| 25% yellow/brown | 3 |
| 25-50% yellow/brown | 2 |
| 75% yellow/brown | 1 |
| 100% yellow/brown | 0 |

**[Table 2-3]**

| (III) Damage Border | |
|---|---|
| Not visible | 4 |
| 25% of border visible | 3 |
| 50% of border visible | 2 |
| 75% of border visible | 1 |
| Entire border visible | 0 |

**[Table 2-4]**

| (IV) Surface | |
|---|---|
| Smooth/normal | 4 |
| Smooth with projections | 3 |
| 25-50% irregular surface | 2 |
| 50-75% irregular surface | 1 |
| >75% irregular surface | 0 |

### (2) Histological Evaluation Method

Repaired tissue was fixed for 1 day in phosphate buffer solution (4%) containing paraformaldehyde and decalcified for 4 weeks with 10% EDTA (Nacalai Tesque, Inc., Kyoto, Japan) followed by embedded in a paraffin block. Samples were prepared by cutting 5 µm sections into a sagittal shape. The sections were stained with safranin O/fast green stain (Muto Pure Chemicals Co., Ltd., Japan) and then subjected to histological scoring according to the Sellers scale for the purpose of histological evaluation (Table 3) [8]. Cell density of the repaired tissue was evaluated using hematoxylin and eosin (H & E) stain.

Table 3 Sellers Scale for Histological Evaluation of Repaired Tissue

**[Table 3-1]**

| (1) Replenishment of Damaged Site relative to Surface of Normal Adjacent Cartilage | |
|---|---|
| 111%-125% | 1 |
| 91%-110% | 0 |
| 76%-90% | 1 |
| 51%-75% | 2 |
| 26%-50% | 3 |
| <25% | 4 |

**[Table 3-2]**

| (2) Integration of Repaired Tissue and Peripheral Joint Cartilage | |
|---|---|
| Normal continuity and integrity | 0 |
| Decreased cellularity | 1 |
| Unilateral gap or lack of continuity | 2 |
| Bilateral gap or lack of continuity | 3 |

**[Table 3-3]**

| (3) Matrix Staining by Safranin O/Fast Green Stain | |
|---|---|
| Normal | 0 |
| Somewhat reduced | 1 |
| Moderately reduced | 2 |
| Substantially reduced | 3 |
| Not stained | 4 |

**[Table 3-4]**

| (4) Cell Morphology ((a)-(d) selected initially) | |
|---|---|
| (a) Normal | 0 |
| (b) Nearly all circular cells have morphology of chondrocytes | |
| • >75% of tissue has columns in zona radiate | 0 |
| • 25%-75% of tissue has columns in zona radiate | 1 |
| • <25% of tissue has columns in zona radiate (disorderly) | 2 |
| (c) 50% of circular cells have morphology of chondrocytes | |
| • >75% of tissue has columns in zona radiate | 2 |
| • 25%-75% of tissue has columns in zona radiate | 3 |
| • <25% of tissue has columns in zona radiate (disorderly) | 4 |
| (d) Nearly all spindle-shaped (fibroblast-like) cells | 5 |

**[Table 3-5]**

| (5) Damage Internal Structure (not including border) | |
|---|---|
| Normal | 0 |
| 1-3 small voids | 1 |
| 1-3 large voids | 2 |
| >3 large voids | 3 |
| Tears or fibrillation | 4 |

**[Table 3-6]**

| (6) Surface Structure | |
|---|---|
| Normal | 0 |
| Slight fibrillation or irregularity | 1 |
| Moderate fibrillation or irregularity | 2 |
| Severe fibrillation or collapse | 3 |

**[Table 3-7]**

| (7) Percentage of New Subchondral Bone | |
|---|---|
| 90%-100% | 0 |
| 75%-89% | 1 |
| 50%-74% | 2 |
| 25%-49& | 3 |
| <25% | 4 |

**[Table 3-8]**

| (8) Formation of Calcification Front | |
|---|---|
| Complete formation | 0 |
| 75%-99% | 1 |
| 50%-74% | 2 |
| 25%-49% | 3 |
| <25% | 4 |

### EXAMPLE 4: Immunostaining

Sections were pretreated with antigen recovery reagent (Immunoactive, Matsunami Glass Ind., Osaka, Japan) at 4 weeks and 12 weeks after treatment and immersed in 0.3% H₂O₂ to block intrinsic peroxidase activity. The sections were then blocked with blocking solution (Protein Block Serum-Free, Dako, Carpinteria, CA) and incubated with mouse monoclonal antibody to type I collagen (1:250, Daiichi Fine Chemical, Toyama, Japan) and type II collagen (1:250, Daiichi Fine Chemical). The visualization reaction was carried out a using avidin-biotin peroxidase system (Vectastain Elite ABC Kit, Vector Laboratories, Inc., Burlingame, CA) followed by staining the sections using 3,3'-diaminobenzidine (Peroxidase Substrate Kit, Vector Laboratories, Inc.).

### EXAMPLE 5: Experiment Results

### (1) Macroscopic Findings

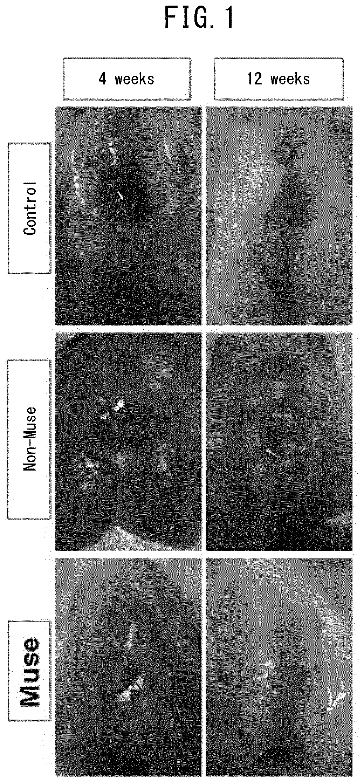

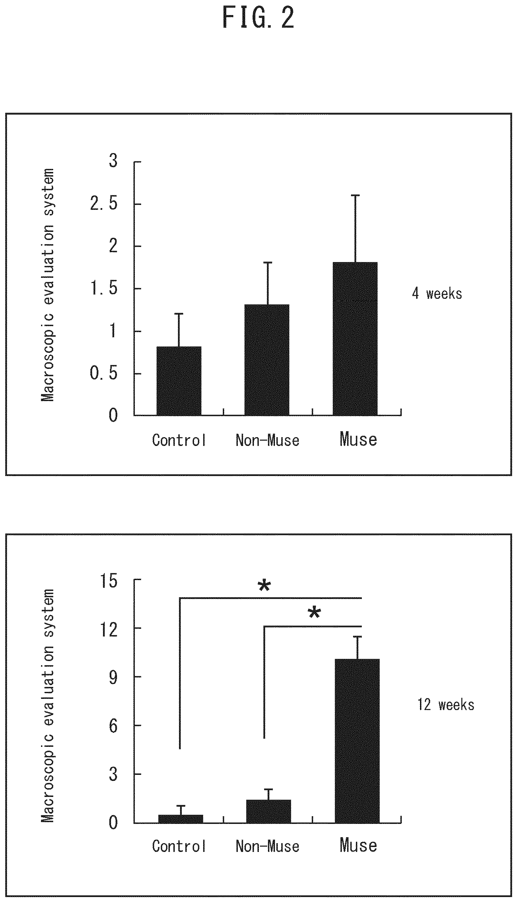

Damage borders were easily identified in the patellar grooves of the control and Muse group at 4 weeks and repaired tissue was not detected. Osteoarthritic changes accompanying degeneration of adjacent cartilage increased in the control group in comparison with the Muse group and non-Muse group at 4 weeks and 12 weeks. At 12 weeks, the depth of the damaged portion was filled with brown tissue and decreased in the non-Muse group. In the Muse group, the damaged portion was completely filled with white tissue, this was smooth corresponding to the surrounding tissue, had a uniform surface and it was difficult to clearly identify the damage border (FIG. 1). Although there were no significant differences in macroscopic scoring between the three groups at 4 weeks after treatment, an improving trend was observed in the control group, non-Muse group and Muse group in that order (control group: 0.8±0.4, non-Muse group: 1.3±0.5, Muse group: 1.8±0.8). However, macroscopic results in the Muse group were significantly better than the results of the other groups following treatment in week 12 when measured according to defect replenishment (control group: 0.5±0.6, non-Muse group: 1.5±0.5, Muse group: 10.0±1.5) (FIG. 2). The macroscopic scores for each parameter are shown in Table 4.

### [Table 4-1]

**Table 4 Macroscopic Scores**

| Coverage | | | | | |
|---|---|---|---|---|---|
| 4 Weeks | | | 12 Weeks | | |
| Control | Non-Muse | Muse | Control | Non-Muse | Muse |
| 0.8±0.4 | 1.3±0.5 | 1.8±0.8* | 0.5±0.6 | 1.5±0.5* | 3.5±0.5* |

**[Table 4-2]**

| Color of New Cartilage | | | | | |
|---|---|---|---|---|---|
| 4 Weeks | | | 12 Weeks | | |
| Control | Non-Muse | Muse | Control | Non-Muse | Muse |
| 0±0 | 0±0 | 0±0 | 0±0 | 0±0 | 2.2±0.8* |

**[Table 4-3]**

| Damage Border | | | | | |
|---|---|---|---|---|---|
| 4 Weeks | | | 12 Weeks | | |
| Control | Non-Muse | Muse | Control | Non-Muse | Muse |
| 0±0 | 0±0 | 0±0 | 0±0 | 0±0 | 2.3±0.5* |

**[Table 4-4]**

| Surface | | | | | |
|---|---|---|---|---|---|
| 4 Weeks | | | 12 Weeks | | |
| Control | Non-Muse | Muse | Control | Non-Muse | Muse |
| 0±0 | 0±0 | 0±0 | 0±0 | 0±0 | 2.0±0* |

| | | | | | |
|---|---|---|---|---|---|
| *: Significantly better in comparison with control group (p<0.05) | | | | | |

### (2) Histological Findings and Scoring

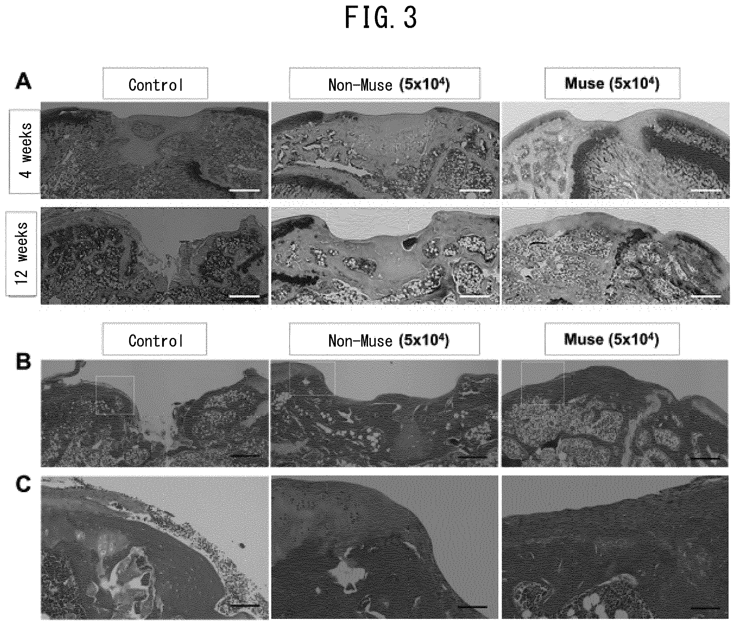

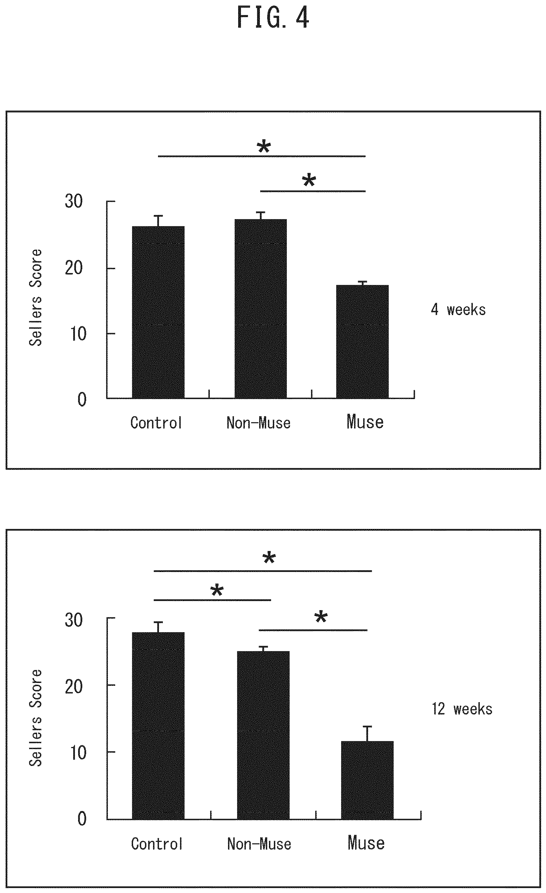

Although there was a small amount of fibrous tissue when animals of the control group and non-Muse group were sacrificed at 4 weeks and 12 weeks, repaired tissue was not observed at the damaged site. There was no cartilage replacement and partial repair of damage accompanying repair of subchondral bone was observed in the Muse group at 4 weeks. In the Muse group at 12 weeks, repair of cartilage damage was confirmed and although this was accompanied by complete repair of subchondral bone, the subchondral bone was covered with fibrous tissue, and integration in addition to osteochondral joining was confirmed (FIG. 3A). The following results were recorded based on the Sellers scale at 4 weeks and 12 weeks, respectively: control group (4 weeks: 26.2±1.6, 12 weeks: 27.8±1.5), non-Muse group (4 weeks: 27.2±1.2, 12 weeks: 25±0.6) and Muse group (4 weeks: 17.4±0.6, 12 weeks: 11.8±2.0). The non-Muse group demonstrated significantly better results than the control group at 12 weeks after treatment. The scores in the Muse group were significantly better than the other groups at both 4 weeks and 12 weeks after treatment (FIG. 4). The Sellers scores for each parameter are shown in Table 5. H & E staining at 12 weeks indicated that cell density of repaired tissue in the Muse group was higher in comparison with the other groups (FIGS. 3B, C).

Table 5 Sellers Scores

**[Table 5-1]**

| Defect Replenishment relative to Surface of Adjacent Cartilage | | | | | |
|---|---|---|---|---|---|
| 4 Weeks | | | 12 Weeks | | |
| Control | Non-Muse | Muse | Control | Non-Muse | Muse |
| 2.0±0.7 | 2.0±0 | 1.2±0.4* | 2.0±0.8 | 2.2±0.4 | 1.0±1.1* |

**[Table 5-2]**

| Integration of Repaired Tissue and Peripheral Joint Cartilage | | | | | |
|---|---|---|---|---|---|
| 4 Weeks | | | 12 Weeks | | |
| Control | Non-Muse | Muse | Control | Non-Muse | Muse |
| 3.0±0 | 3.0±0 | 3.0±0 | 3.0±0 | 3.0±0 | 0±0* |

**[Table 5-3]**

| Matrix Staining by Safranin O/Fast Green Stain | | | | | |
|---|---|---|---|---|---|
| 4 Weeks | | | 12 Weeks | | |
| Control | Non-Muse | Muse | Control | Non-Muse | Muse |
| 4.0±0 | 4.0±0 | 4.0±0 | 4.0±0 | 4.0±0 | 4±0 |

**[Table 5-4]**

| Cell Morphology ((a)-(d) selected initially) | | | | | |
|---|---|---|---|---|---|
| 4 Weeks | | | 12 Weeks | | |
| Control | Non-Muse | Muse | Control | Non-Muse | Muse |
| 5.0±0 | 5.0+0 | 5.0±0 | 5.0±0 | 5.0±0 | 5±0 |

**[Table 5-5]**

| Damage Internal Structure (not including border) | | | | | |
|---|---|---|---|---|---|
| 4 Weeks | | | 12 Weeks | | |
| Control | Non-Muse | Muse | Control | Non-Muse | Muse |
| 2.8±1.3 | 3.5±1.2 | 0.2±0.4* | 3.0±1.1 | 1.0±0* | 0±0* |

**[Table 5-6]**

| Surface Structure | | | | | |
|---|---|---|---|---|---|
| 4 Weeks | | | 12 Weeks | | |
| Control | Non-Muse | Muse | Control | Non-Muse | Muse |
| 1.6±0.9 | 2.0±0.6 | 1.2±0.4 | 2.8±0.5 | 3.0±0 | 1.0±0* |

**[Table 5-7]**

| Percentage of New Subchondral Bone | | | | | |
|---|---|---|---|---|---|
| 4 Weeks | | | 12 Weeks | | |
| Control | Non-Muse | Muse | Control | Non-Muse | Muse |
| 3.8±0.4 | 4.0±0 | 1.0±0.7* | 4.0±0 | 3.0±0* | 0.3±0.5* |

**[Table 5-8]**

| Formation of Calcification Front | | | | | |
|---|---|---|---|---|---|
| 4 Weeks | | | 12 Weeks | | |
| Control | Non-Muse | Muse | Control | Non-Muse | Muse |
| 4.0±0 | 4.0±0 | 1.8±1.1* | 4.0±0 | 3.8±0.4 | 0.5±0.5* |

Intraarticular injection of Muse cells is a promising method for repairing osteochondral damage, and particularly subchondral bone covered by fibrous tissue.
[1] A. Mobasheri, C. Csaki, A.L. Clutterbuck, M. Rahmanzadeh, and M. Shakibaei, Mesenchymal stem cells in connective tissue engineering and regenerative medicine: applications in cartilage repair and osteoarthritis therapy. Histol Histopathol, vol. 24, no. 3, pp. 347-366, 2009.
[2] M. Brittberg, A. Lindahl, A. Nilsson, C. Ohlsson, O. Isaksson, and L. Peterson, Treatment of deep cartilage defects in the knee with autologous chondrocyte transplantation. N Engl J Med, vol. 331, no. 14, pp. 889-895, 1994.
[3] M. Ochi, Y. Uchio, K. Kawasaki, S. Wakitani, and J. Iwasa, Transplantation of cartilage-like tissue made by tissue engineering in the treatment of cartilage defects of the knee. J Bone Joint Surg Br, vol. 84, no. 4, pp. 571-578, 2002.
[4] K. Tamai, T. Yamazaki, T. Chino, et al., PDGFRalpha-positive cells in bone marrow are mobilized by high mobility group box 1 (HMGB1) to regenerate injured epithelia. Proc Natl Acad Sci U S A, vol. 108, no. 16, pp. 6609-6614, 2011.
[5] Y. Kuroda, S. Wakao, M. Kitada, T. Murakami, M. Nojima, and M. Dezawa, Isolation, culture and evaluation of multilineage-differentiating stress-enduring (Muse) cells. Nat Protoc, vol. 8, no. 7, pp. 1391-1415, 2013.
[6] J.S. Wayne, C.L. McDowell, K.J. Shields, and R.S. Tuan, In vivo response of polylactic acid-alginate scaffolds and bone marrow-derived cells for cartilage tissue engineering. Tissue Eng, vol. 11, no. 5-6, pp. 953-963, 2005.
[7] W. Cui, Q. Wang, G. Chen, et al., Repair of articular cartilage defects with tissue-engineered osteochondral composites in pigs. J Biosci Bioeng, vol. 111, no. 4, pp. 493-500, 2011.
[8] R.S. Sellers, D. Peluso, and E.A. Morris, The effect of recombinant human bone morphogenetic protein-2 (rhBMP-2) on the healing of full-thickness defects of articular cartilage. J Bone Joint Surg Am, vol. 79, no. 10, pp. 1452-1463, 1997.

## Claims

1. A cell preparation for treating or repairing osteochondral damage, comprising: pluripotent stem cells positive for SSEA-3 isolated from a body mesenchymal tissue or cultured mesenchymal cells.

2. The cell preparation according to claim 1, wherein the cell preparation contains a cell fraction wherein the pluripotent stem cells positive for SSEA-3 have been concentrated by external stress stimulation.

3. The cell preparation according to claim 1 or 2, wherein the pluripotent stem cells are CD105-positive.

4. The cell preparation according to any one of claims 1 to 3, wherein the pluripotent stem cells are CD117-negative and CD146-negative.

5. The cell preparation according to any one of claims 1 to 4, wherein the pluripotent stem cells are CD117-negative, CD146-negative, NG2-negative, CD34-negative, vWF-negative and CD271-negative.

6. The cell preparation according to any one of claims 1 to 5, wherein the pluripotent stem cells are CD34-negative, CD117-negative, CD146-negative, CD271-negative, NG2-negative, vWF-negative, Sox10-negative, Snail-negative, Slug-negative, Tyrp1-negative and Dct-negative.

7. The cell preparation according to any one of claims 1 to 6, wherein the pluripotent stem cells are pluripotent stem cells having all of the properties indicated below:
(i) low or absent telomerase activity;
(ii) ability to differentiate into any of three germ layers;
(iii) absence of demonstration of neoplastic proliferation; and,
(iv) self-renewal ability.

8. The cell preparation according to any one of claims 1 to 7, wherein the pluripotent stem cells have the ability to accumulate at sites of osteochondral damage.

9. The cell preparation according to any one of claims 1 to 8, wherein the pluripotent stem cells have the ability to differentiate into chondrocytes.
